(19) Europäisches Patentamt
European Patent Office
Office européen des brevets

(11) Publication number: 0 300 527 B1

# (12) EUROPEAN PATENT SPECIFICATION

(45) Date of publication of patent specification: 04.12.91 Bulletin 91/49

(51) Int. Cl.$^5$: C07C 43/11, C07C 43/13, C07C 41/16

(21) Application number: 88201296.6

(22) Date of filing: 23.06.88

## (54) Process for synthetizing polyoxyalkyleneglycol-mono-ethers.

(30) Priority: 24.07.87 IT 2143287

(43) Date of publication of application: 25.01.89 Bulletin 89/04

(45) Publication of the grant of the patent: 04.12.91 Bulletin 91/49

(84) Designated Contracting States: AT BE CH DE ES FR GB GR LI LU NL SE

(56) References cited:
EP-A- 0 021 497
DE-A- 3 025 807
GB-A- 1 178 750

(56) References cited:
FETTE, SEIFEN, ANSTRICHMITTEL, 74, 1972, Industrieverlag von Hernhaussen KG, Hamburg, DE; Y. ABE et al.: "Synthesis of Decyl, Dodecyl, Tetradecyl and Nonylphenyl Mono-ethers of Hexato Nonaethylene: glycol"

(73) Proprietor: ENIRICERCHE S.p.A.
Corso Venezia 16
I-20121 Milan (IT)

(72) Inventor: Tinucci, Laura
Via Lazzarini 5
I-20060 Cervignano d'Adda Milan (IT)

(74) Representative: Roggero, Sergio et al
Ing. Barzanò & Zanardo Milano S.p.A. Via Borgonuovo 10
I-20121 Milano (IT)

EP 0 300 527 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for synthetizing polyoxyalkyleneglycol-monoethers and to the products obtained by means of such a process, which find important uses as non-ionic surfactants in the cosmetic and/or pharmaceutical field.

The synthesis of polyoxyalkyleneglycol-monoethers containing a predetermined number of oxyalkylene units is a target which the chemistry of surfactants aimed at, in order to obtain non-ionic surfactants endowed with controllable properties.

The prior art relating to non-ionic surfactants of ethoxylated type carries out the ethoxylation of the feedstock molecule by means of ethylene oxide, but in this way a random distribution of ethylene oxide units is obtained, and the product only containing the desired number of ethylene oxide cannot be obtained as the only product.

In the field of the synthesis of polyoxyalkyleneglycol-monoethers, the present Applicant is aware of the works by Y. Abe and S. Watanabe (Fette, Seifen, Anstrichtmittel, 74, No. 9, 1972, pages 534-537).

According to the procedure followed by Abe and Watanabe, an alcohol or a phenol is reacted with potassium metal with the consequent formation of the corresponding potassium alkoxide or phenate. This latter is then reacted with mono-chloro oxyalkylene-glycol, with the monoether being obtained.

The procedure followed is clearly unsuitable for an industrial production, in that metal potassium is not easy to handle from an industrial standpoint.

The present Applicant has surprisingly found now, and this is the object of the present invention, that polyoxyalkyleneglycol-monoethers can be prepared in a simple and cheap way, with the drawbacks of the prior art being overcome, by using mono-halo-oxyethylene-glycols with the halogen in a terminal position, and the hydroxy group being protected by known means.

A first object of the present invention is a process for the synthesis of polyoxyalkyleneglycol-monoethers which comprises reacting a linear or branched aliphatic alcohol, preferably a primary linear aliphatic alcohol or a — preferably alkyl-substituted or alkoxy-substituted — mono-hydroxy-phenol, having the general formula

$$R\text{-}OH \quad (I)$$

wherein :

R is an alkyl radical containing a number of carbon atoms comprised within the range of from 6 to 20, or a phenyl radical, preferably bearing alkyl-substituents or alkoxy-substituents with a number of carbon atoms comprised within the range of from 1 to 10, or the product of general formula (4), with a linear mono-halo-oxyalkylene glycol having the residual hydroxy group protected by means of a protecting agent, having the general formula

$$Ha\text{-}(CH_2)_n\text{-}(CH_2\text{-}O\text{-}CH_2)_m\text{-}(CH_2)_n\text{-}O\text{-}PR \quad (2)$$

wherein :

Ha is a halogen selected from Cl, Br, I ;
PR is a protecting group for the terminal hydroxy ; and
n and m are integers, either equal to, or different from, each other, having values respectively comprised within the range of from 1 to 2 and of from 1 to 5.

The protecting group PR is a hydroxy-protecting group and is selected in particular from
— tetrahydropyranyl,
— 4-methoxy-tetrahydro-pyranyl,
— 4-methoxy-tetrahydro-thio-pyranyl
groups, respectively deriving from :
— 3,4-dihydro-2H-pyran,
— 4-methoxy-2,3-dihydro-6H-pyran and
— 4-methoxy-2,3-dihydro-thio-6H-pyran.

The reaction is carried out in the liquid phase in the presence of a base preferably selected from NaOH and KOH.

The reaction product, having the general formula

$$R\text{-}O\text{-}(CH_2)_n\text{-}(CH_2\text{-}O\text{-}CH_2)_m\text{-}(CH_2)_n\text{-}O\text{-}PR \quad (3)$$

wherein n and m have the above stated values, is submitted to an alcoholysis at room temperature under acidic conditions, preferably with methanol, and an acidic ion-exchange resin, with said resin preferably, but not limitedly, being a sulphonated styrene-divinylbenzene resin, in particular the commercial resins known on the market under the trade marks Amberlyst 15 by Rohm and Haas and Lewatit P108 by Bayer.

The de-protected product according to the present invention, having the general formula

$$R\text{-}O\text{-}(CH_2)_n\text{-}(CH_2\text{-}O\text{-}CH_2)_m\text{-}(CH_2)_n\text{-}O\ H \quad (4)$$

wherein n and m have the above stated values, is filtered off from the resin and can be used as surfactant according to the invention or, if so preferred, can be used as the raw material instead of said compound of formula (1) if the number of oxyalkylene glycol units has to be further increased.

The temperature at which the reaction between the compound of general formula (1) and the compound of general formula (2) is carried out is preferably selected within the range of from 80°C to 110°C, and the pressure is preferably the atmospheric pressure. The base is preferably used in an equimolecular amount relatively to the compound of general formula (2).

The number of $H^+$ milliequivalents of the resin used in the step of removal of the protecting group is equal to that of the compound of formula (3), but a lower number, down to half thereof, can be used as well.

In order to obtain the product having the general formula (2), the procedure followed consists in reacting under anhydrous conditions, in the presence of a halogenated solvent, at a temperature comprised within the range of from 10°C to 50°C, and preferably at a temperature comprised within the range of from 20°C to 40°C, a monohalogenated alkoxy-glycol (formula (2) with a terminal —OH in place of the —OPR group) with a hydroxy-protecting agent preferably selected from :

— 3,4-dihydro-2H-pyran,
— 4-methoxy-2,3-dihydro-6H-pyran, and
— 4-methoxy-2,3-dihydro-thio-6H-pyran,

for a time comprised within the range of from 1 minute to 30 minutes, and in distilling the protected compound (2) of from the halogenated solvent.

Some examples are given now in order to better explain the invention, it being understood that the same invention is not to be construed as being limited by them or to them.

## Example 1

Synthesis of 2-(2-Chloroethoxy)-tetrahydropyranyl-ethanol (Compound of general formula (2))

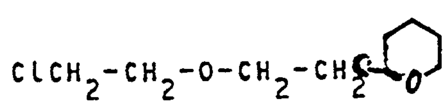

To a reaction flask containing 15 g (120 mmol) of 2-(2-chloro-ethoxy)-ethanol dissolved in 30 ml of methylene chloride, 12 g (143 mmol) of 3,4-dihydro-2H-pyran dissolved in 25 ml of methylene chloride is added dropwise (addition time = 30 minutes). The reaction is exothermal (initial temperature = 17°C, end temperature = 37°C) and results practically complete after 10 minutes of the end of the addition.

The raw reaction product, concentrated under reduced pressure supplies, after distillation, 23 g (yield 92%) of chemically pure 2-(2-chloroethoxy)-tetrahydro-pyranyl-ethanol.

## Example 2

Synthesis of [2- 2-(2-Chloroethoxy)-ethoxy]-tetrahydropyranyl-ethanol (Compound of general formula (2))

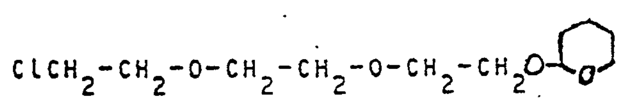

The reaction is carried out at room temperature by a procedure analogous to that as reported for the synthesis of 2-(2-chloroethoxy)-tetrahydro-pyranyl-ethanol, and is complete after 30 minutes of the end of the addition of 3,4-dihydro-2H-pyran.

By starting from 105 g (623 mmol) of 2-[2-(2-chloroethoxyl-ethoxy]-ethanol (dissolved in 150 ml of methylene chloride) and from 63 g (750 mmol) of 3,4-dihydro-2H-pyran (dissolved in 100 ml of methylene chloride), after distillation 135 g of chemically pure 2-[2-(2-chloroethoxy)-ethoxy]-tetrahydro-pyranyl-ethanol were obtained (yield 86%).

Example 3

Synthesis of dioxyethyleneglycol-monododecylether

$$CH_3-(CH_2)_{10}-CH_2-O-CH_2-CH_2-O-CH_2-CH_2-OH$$

A mixture of 16.80 g (80 mmol) of 2-(2-chloroethoxy)-tetrahydropyranyl-ethanol, 3.22 g (80 mmol) of sodium hydroxide powder and 0.36 cc of water is dipped, while is being mechanically stirred, into an oil bath (pre-heated at 115°C) and to it 5.01 g (27 mmol) of 1-dodecanol is added dropwise (addition time = 10 minutes).

Eight hours later, the reaction mixture is cooled to room temperature, to it 20 ml of water and 200 ml of ethyl acetate are added. The organic phase is separated from the aqueous phase and this latter is extracted with a further 100 ml of ethyl acetate. The combined organic extracts are dried over anhydrous sodium sulphate, are concentrated under reduced pressure, are then diluted with methyl alcohol (50 ml) and to them 4 g of Amberlyst 15 resin is added. After a 1-hour stirring at room temperature, the suspension is filtered, concentrated under reduced pressure and is distilled ; 3.70 g of product is obtained (yield 50%).

Example 4

Synthesis of tetraoxyethyleneglycol-monododecylether

$$CH_3(CH_2)_{10}-CH_2-O-CH_2CH_2-O-CH_2CH_2-O-CH_2CH_2-O-CH_2CH_2-OH$$

The reaction was carried out on 6.85 g (33 mmol) of 2-(2-chloroethoxy)-tetrahydropyranyl-ethanol, 1.31 g (33 mmol) of sodium hydroxide powder, 0.16 ml of water and 3.00 g (11 mmol) of dioxythyleneglycol-monododecylether.

After a 4-hour heating at 90°C, the reaction product is hydrolyzed and extracted with ethyl acetate as usual. The organic extracts are combined, dried over anhydrous sodium sulphate, concentrated under reduced pressure and to them 2.34 g of Amberlyst 15 resin is added, after being preliminarily diluted with 50 ml of methyl alcohol. After 2 hours at room temperature, the suspension is filtered, concentrated under reduced pressure and distilled ; 2.80 g (yield 70%) of desired product is obtained.

In an analogous reaction carried out by starting from a mixture consisting of 10 g (48 mmol) of 2-(2-chloroethoxy)-tetrahydropyranyl-ethanol, 1.92 g (48 mmol) of sodium hydroxide powder, and 4.38 g (16 mmol) of dioxythyleneglycol-monododecylether, 4.34 g of tetraoxyethyleneglycol-monododecylether was obtained (yield 75%).

Example 5

Synthesis of hexaoxyethyleneglycol-monododecylether

$$CH_3-(CH_2)_{10}-CH_2-(O-CH_2-CH_2)_6-OH$$

The reaction was carried out on 8.76 g (42 mmol) of 2-(2-chloroethoxy)-tetrahydropyranyl-ethanol, 1.68 g (42 mmol) of sodium hydroxide powder, 0.19 ml of water and 5.00 g (14 mmol) of tetraoxyethyleneglycol-monododecylether. After 5 hours of heating at 110°C, the mixture was hydrolyzed and extracted with ethyl acetate, as usual.

The combined organic extracts, after being dried over anhydrous sodium sulphate and concentrated under reduced pressure, were diluted with methyl alcohol (60 ml) and to them 4 g of Amberlyst 15 resin was added.

After 2 hours at room temperature, the suspension was filtered, and concentrated under reduced pressure; 47 g of hexaoxyethyleneglycol-monododecylether (yield 75%) was obtained.

Example 6

Synthesis of hexaoxyethyleneglycol-monooctylether

$$CH_3\text{-}(CH_2)_6\text{-}CH_2\text{-}(O\text{-}CH_2\text{-}CH_2)_6\text{-}OH$$

The reaction was carried out on 10.22 g (49 mmol) of 2-(2-chloroethoxy)-tetrahydropyranyl-ethanol, 1.96 g (49 mmol) of sodium hydroxide powder, 0.2 ml of water and 5.00 g (16 mmol) of tetraoxyethyleneglycol-monooctylether.

After 7 hours of heating, the mixture was treated as hereinabove reported. The combined organic extracts were diluted with methyl alcohol (70 ml), to them 4 g of Amberlyst 15 resin was added, and the whole mixture was kept stirred at room temperature for 2 hours. It was then filtered, concentrated under reduced pressure and distilled. 4.6 g (yield 73%) of the desired product was obtained.

## Claims

1. Process for the synthesis of polyoxyalkyleneglycol-monoethers by starting from a linear or branched aliphatic alcohol, preferably a primary linear aliphatic alcohol or a mono-hydroxy-phenol, preferably alkyl-substituted or alkoxy-substituted, having the general formula

$$R\text{-}OH \quad (I)$$

wherein :

R is an alkyl radical containing a number of carbon atoms comprised within the range of from 6 to 20, or a phenyl radical, preferably bearing alkyl-substituents or alkoxy-substituents with a number of carbon atoms comprised within the range of from 1 to 10,
characterized in that the product of formula (1) is reacted with a linear oxyalkylene glycol mono-halogenated in a terminal position, having the residual hydroxy group protected by means of a protecting agent, having the general formula

$$Ha\text{-}(CH_2)_n\text{-}(CH_2\text{-}O\text{-}CH_2)_m\text{-}(CH_2)_n\text{-}O\text{-}PR \quad (2)$$

wherein :

Ha is a halogen selected from Cl, Br, I ;
PR is a protecting group ; and
n and m are integers having values respectively comprised within the range of from 1 to 2 and of from 1 to 5, the reaction being carried out in the liquid phase in the presence of a base, the reaction product, having the general formula

$$R\text{-}O\text{-}(CH_2)_n\text{-}(CH_2\text{-}O\text{-}CH_2)_m\text{-}(CH_2)_n\text{-}O\text{-}PR \quad (3)$$

wherein n and m have the above stated values, is submitted to an alcoholysis at room temperature under acidic conditions, with methanol, and an acidic ion-exchange resin, and the de-protected product having the general formula

$$R\text{-}O\text{-}(CH_2)_n\text{-}(CH_2\text{-}O\text{-}CH_2)_m\text{-}(CH_2)_n\text{-}O\ H \quad (4)$$

wherein n and m have the above stated values, is filtered off from the resin.

2. Process according to claim 1, characterized in that PR in the above general formula (2) represents a hydroxy-protecting group and is selected in particular from
— tetrahydropyranyl,
— 4-methoxy-tetrahydro-thio-pyranyl groups
— 4-methoxy-tetrahydro-pyranyl.

3. Process according to claim 1, characterized in that the base is in an equimolecular amount relatively to the compound of general formula (2).

4. Process according to claim 1 or 3, characterized in that the base is selected from KOH and NaOH.

5. Process according to claim 1, characterized in that the acidic ion-exchange resin has a number of $H^+$ milliequivalents equal to that of the compound of formula (3), or lower than that, down to the half.

6. Process according to claim 1 or 5, characterized in that the acidic ion-exchange resin is a sulphonated styrene-divinyl-benzene resin.

7. Process according to claim 1, characterized in that the reaction of the compound of general formula (1) with the compound of general formula (2) is carried out at a temperature comprised within the range of from 80°C to 110°C, with the pressure preferably being the atmospheric pressure.

8. Process according to claim 1, characterized in that the compound of general formula (4) replaces the compound of general formula (1) in the reaction with the compound of general formula (2).

9. Process according to claim 1, characterized in that the product having the general formula (2) is obtained by reacting under anhydrous conditions, in the presence of a halogenated solvent, at a temperature comprised within the range of from 10°C to 50°C, and preferably at a temperature comprised within the range of from 20°C to 40°C, a halogenated alkoxy-glycol of formula

$$Ha\text{-}(CH_2)_n\text{-}(CH_2\text{-}O\text{-}CH_2)_n\text{-}(CH_2)_n\text{-}OH$$

wherein n and m have the above stated values, with a hydroxy-protecting agent for a time comprised within the range of from 1 minute to 30 minutes, and distilling the compound (2) off from the halogenated solvent.

10. Process according to claim 9, characterized in that the hydroxy-protecting agent is selected from
— 3,4-dihydro-2H-pyran,
— 4-methoxy-2,3-dihydro-6H-pyran and
— 4-methoxy-2,3-dihydro-thio-6H-pyran.

## Revendications

1. Procédé de synthèse de monoéthers de poly(oxyalkylène-glycol) à partir d'un alcool aliphatique linéaire ou ramifié, de préférence un alcool aliphatique linéaire primaire, ou d'un phénol monohydroxylé, de préférence à substituant alkyle ou alcoxy, qui présente la formule générale

$$R\text{-}OH \quad (I)$$

dans laquelle :

R représente un radical alkyle comportant un nombre d'atomes de carbone compris dans l'intervalle allant de 6 à 20, ou un radical phényle, portant de préférence des substituants alkyle ou des substituants alcoxy comportant un nombre d'atomes de carbone compris dans l'intervalle allant de 1 à 10,

**caractérisé** en ce que l'on fait réagir le produit de formule (I) avec un oxyalkylène-glycol linéaire monohalogéné en position terminale, dont le groupe hydroxy restant est protégé au moyen d'un agent protecteur, et qui présente la formule générale :

$$Ha\text{-}(CH_2)_n\text{-}(CH_2\text{-}O\text{-}CH_2)_m\text{-}(CH_2)_n\text{-}O\text{-}PR \quad (2)$$

dans laquelle :

Ha représente un atome d'halogène choisi parmi Cl, Br et I,
PR représente un groupe protecteur, et,
n et m représentent des nombres entiers dont les valeurs sont respectivement comprises dans les intervalles allant de 1 à 2 et de 1 à 5,

La réaction est effectuée en phase liquide en présence d'une base, et le produit de réaction, qui présente la formule générale

$$R\text{-}O\text{-}(CH_2)_n\text{-}(CH_2\text{-}O\text{-}CH_2)_m\text{-}(CH_2)_n\text{-}O\text{-}PR \quad (3)$$

dans laquelle n et m ont les valeurs indiquées ci-dessus, est soumis à une alcoolyse à température ambiante

dans des conditions acides, avec du méthanol et une résine échangeuse d'ions de type acide, et le produit déprotégé, qui présente la formule générale

$$R\text{-}O\text{-}(CH_2)_n\text{-}(CH_2\text{-}O\text{-}CH_2)_m\text{-}(CH_2)_n\text{-}OH \quad (4)$$

dans laquelle n et m ont les valeurs indiquées ci-dessus, est séparé par filtration d'avec la résine.

2. Procédé conforme à la revendication 1, **caractérisé** en ce que PR, dans la formule générale (2) indiquée ci-dessus, représente un groupe hydroxy-protecteur et est choisi en particulier parmi les groupes tétrahydropyranyle, 4-méthoxy-tétrahydro-thio-pyranyle et 4-méthoxy-tétrahydro-pyranyle.

3. Procédé conforme à la revendication 1, **caractérisé** en ce que la base est présente en une quantité équimoléculaire par rapport au composé de formule générale (2).

4. Procédé conforme à la revendication 1 ou 3, **caractérisé** en ce que la base est choisie parmi KOH et NaOH.

5. Procédé conforme à la revendication 1, **caractérisé** en ce que la résine échangeuse d'ions de type acide présente un nombre de milliéquivalents $H^+$ égal ou inférieur, à celui du composé de formule (3), mais au moins égal à la moitié de ce nombre-ci.

6. Procédé conforme à la revendication 1 ou 5, **caractérisé** en ce que la résine échangeuse d'ions de type acide est une résine de styrène/divinylbenzène sulfonée.

7. Procédé conforme à la revendication 1, **caractérisé** en ce que la réaction du composé de formule générale (1) avec le composé de formule générale (2) est effectuée à une température comprise dans l'intervalle allant de 80°C à 110°C, la pression étant de préférence égale à la pression atmosphérique.

8. Procédé conforme à la revendication 1, **caractérisé** en ce que le composé de formule générale (4) remplace le composé de formule générale (1) dans la réaction avec le composé de formule générale (2).

9. Procédé conforme à la revendication 1, **caractérisé** en ce que le produit présentant la formule générale (2) est obtenu par réaction dans des conditions anhydres, en présence d'un solvant halogéné, à une température comprise dans l'intervalle allant de 10 à 50°C, et de préférence à une température comprise dans l'intervalle allant de 20 à 40°C, d'un alcoxy-glycol halogéné de formule

$$Ha\text{-}(CH_2)_n\text{-}(CH_2\text{-}O\text{-}CH_2)_m\text{-}(CH_2)_n\text{-}OH$$

dans laquelle n et m ont les valeurs indiquées ci-dessus, avec un agent hydroxy-protecteur, pendant une durée comprise dans l'intervalle allant de 1 minute à 30 minutes, et séparation par distillation du composé (2) d'avec le solvant halogéné.

10. Procédé conforme à la revendication 9, **caractérisé** en ce que l'agent hydroxy-protecteur est choisi parmi le 3,4-dihydro-2H-pyrane, le 4-méthoxy-2,3-dihydro-6H-pyrane et le 4-méthoxy-2,3-dihydro-thio-6H-pyrane.

**Patentansprüche**

1. Verfahren zur Herstellung von Polyoxyalkylenglykol-monoethern, ausgehend von einem linearen oder verzweigten aliphatischen Alkohol, vorzugsweise einem primären linearen aliphatischen Alkohol, oder von einem Monohydroxyphenol, vorzugsweise einem Alkyl- oder Alkoxy-substituierten Monohydroxyphenol, mit der allgemeinen Formel

$$R\text{-}OH \quad (1),$$

worin :

R für einen Alkylrest mit einer Kohlenstoffatomanzahl im Bereich von 6 bis 20 oder für einen Phenylrest steht, der vorzugsweise Alkyl- oder Alkoxysubstituenten mit 1 bis 10 Kohlenstoffatomen trägt, dadurch gekennzeichnet, daß das Produkt der Formel (1) mit einem in einer Endstellung monohalogenierten linearen Oxyalkylenglykol, dessen verbleibende Hydroxygruppe mit einer Schutzgruppe geschützt ist, entsprechend der allgemeinen Formel

$$Ha\text{-}(CH_2)_n\text{-}(CH_2\text{-}O\text{-}CH_2)_m\text{-}(CH_2)_n\text{-}O\text{-}PR \quad (2),$$

worin :

Ha ein unter Cl, Br und I ausgewähltes Halogen ist;
PR **eine Schutzgruppe darstellt; und**
n und m ganze Zahlen mit Werten im Bereich 1 bis 2 bzw. 1 bis 5 bedeuten, umgesetzt wird, wobei die Reaktion in flüssiger Phase im Anwesenheit einer Base ausgeführt wird, daß das Reaktionsprodukt mit der allgemeinen Formel

$$R\text{-}O\text{-}(CH_2)_n\text{-}(CH_2\text{-}O\text{-}CH_2)_m\text{-}(CH_2)_n\text{-}O\text{-}PR \quad (3),$$

worin n und m die vorstehend angegebenen Werte aufweisen, bei Raumtemperatur unter sauren Bedingungen einer Alkoholyse mit Methanol und einem sauren Ionenaustauscherharz unterworfen wird und das von der Schutzgruppe befreite Produkt mit der allgemeinen Formel

$$R\text{-}O\text{-}(CH_2)_n\text{-}(CH_2\text{-}O\text{-}CH_2)_m\text{-}(CH_2)_n\text{-}OH \quad (4),$$

worin n und m die vorstehend angegebenen Werte aufweisen, vom Harz abfiltriert wird.

2. Verfahren nach Anspruch 1, dadurch gekennzeichnet, daß PR in der vorstehenden allgemeinen Formel (2) eine Hydroxyschutzgruppe darstellt und insbesondere unter
— Tetrahydropyranyl,
— 4-Methoxy-tetrahydro-thio-pyranylgruppen und
— 4-Methoxy-tetrahydro-pyranyl
ausgewählt wird.

3. Verfahren nach Anspruch 1, dadurch gekennzeichnet, daß die Base in äquimolarer Menge, bezogen auf die Verbindung der allgemeinen Formel (2), vorliegt.

4. Verfahren nach Anspruch 1 oder 3, dadurch gekennzeichnet, daß die Base unter KOH und NaOH ausgewählt wird.

5. Verfahren nach Anspruch 1, dadurch gekennzeichnet, daß das saure Ionenaustauscherharz eine $H^+$-Milliäquivalentenzahl aufweist, die derjenigen der Verbindung der Formel (3) gleich ist oder unter dieser, herab bis zum Hälftewert, liegt.

6. Verfahren nach Anspruch 1 oder 5, dadurch gekennzeichnet, daß das saure Ionenaustauscherharz ein sulfoniertes Styrol-divinylbenzolharz ist.

7. Verfahren nach Anspruch 1, dadurch gekennzeichnet, daß die Umsetzung der Verbindung der allgemeinen Formel (1) mit der Verbindung der allgemeinen Formel (2) bei einer Temperatur im Bereich von 80°C bis 110°C ausgeführt wird, wobei der Druck vorzugsweise der Atmosphärendruck ist.

8. Verfahren nach Anspruch 1, dadurch gekennzeichnet, daß die Verbindung der allgemeinen Formel (4) die Verbindung der allgemeinen Formel (1) in der Umsetzung mit der Verbindung der allgemeinen Formel (2) ersetzt.

9. Verfahren nach Anspruch 1 dadurch gekennzeichnet, daß das Produkt mit der allgemeinen Formel (2) durch Umsetzen eines halogenierten Alkoxyglykols der Formel

$$Ha\text{-}(CH_2)_n\text{-}(CH_2\text{-}O\text{-}CH_2)_m\text{-}(CH_2)_n\text{-}OH,$$

worin n und m die vorstehend angegebenen Werte aufweisen, mit einem Hydroxylgruppenschutzmittel in Gegenwart eines halogenierten Lösungsmittels bei einer Temperatur im Bereich von 10°C bis 50°C, vorzugsweise bei einer Temperatur im Bereich von 20°C bis 40°C, während einer Zeitdauer im Bereich von 1 Minute bis 30 Minuten und Abdestillieren der Verbindung (2) vom halogenierten Lösungsmittel erhalten wird.

10. Verfahren nach Anspruch 9, dadurch gekennzeichnet, daß das Hydroxygruppenschutzmittel unter
— 3,4-Dihydro-2H-pyran,
— 4-Methoxy-2,3-dihydro-6H-pyran und
— 4-Methoxy-2,3-dihydro-thio-6H-pyran
ausgewählt wird.